# EUROPEAN PATENT APPLICATION

(11) **EP 0 806 194 A1**
(43) Date of publication of application: **12.11.1997**
(21) Application number: 96110574.9
(22) Date of filing: 01.07.1996
(51) Int. Cl.: A61F 13/15

(54) **Disposable absorbent article being capable of self-shaping in use and comprising odour control means**

(30) Priority: 29.04.1996 EP 96106721; 29.04.1996 EP 96106724; 29.04.1996 EP 96106723
(71) Applicant: THE PROCTER & GAMBLE COMPANY, Cincinnati, Ohio 45202 (US)
(72) Inventor: Carlucci, Giovanni, 66100 Chieti (IT); Divo, Michael, 61381 Friedrichsdorf (DE); Corzani, Italo, 66100 Chieti (IT); Giorgini, Gennaro, 64026 Roseto (IT); Marinelli, Luigi, 65129 Pescara (IT)
(74) Representative: Hirsch, Uwe Thomas

(57) **Abstract**

A disposable absorbent article which is substantially flat prior to use for wearing adjacent a body discharge area having a longitudinal centreline and a lateral centreline orthogonal thereto and defining longitudinal and lateral directions respectively. The disposable absorbent article comprises a liquid pervious topsheet, a backsheet joined to said topsheet and an absorbent core intermediate the backsheet and the topsheet. The absorbent core comprises means for expanding the article into a tridimensional structure while being worn by a user. The means is activated by body fluids. The absorbent article further comprises an odour-control material comprising one or more odour-control agents.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to substantially flat disposable absorbent sanitary napkins, catamenials, incontinence inserts, pantiliners and diapers comprising means for expanding the article into a tridimensional structure while being worn by a user. The means is activated by body fluids and provides the article with a self-shaping capability during the use. The article further comprises an odour-control material comprising one or more odour-control agents in order to minimize odour caused by body fluids.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

In addition to the three functional elements mentioned above, disposable absorbent articles are generally provided with means for supporting the device adjacent the user's crotch area, even as the user moves, where it can most effectively perform its intended function. Typically, absorbent articles as sanitary napkins are provided with an adhesive attachment means for securing the device to the inner crotch area of the user's undergarments.

An improvement in the comfort and convenience of such absorbent articles has been in the development of absorbent articles that achieve a better fluid interception by means of a closer contact of the absorbent article itself with the body of the wearer.

Another aspect of absorbent articles that has been under investigation for many years is that of odour control. Many body fluids have an unpleasant odour, or develop such odours when in contact with air and/or bacteria for prolonged periods. The literature shows many references relating to odour control in products such as diapers and catamenials.

With respect to disposable sanitary napkins several attempts have been made in the art to improve body contact with the wearer, and hence absorb fluids upon discharge and thereby minimize soiling by providing a sanitary napkin having an anatomically shaped configuration, particularly including those that are raised upwardly or humped in their medial portions so as to be near or in contact with the pudendal region when worn.

On female users this type of sanitary napkins attempts to contact and absorb menses immediately as it leaves the vestibule.

Some articles have been also described in which an anatomically shaped configuration is provided during the wearing time, with the advantage of a better fit to the anatomy.

In European applications EP 96106721.2, EP 96106723.8 and EP 96106724.6, filed together on 29 April 1996, absorbent articles are described which comprise means for expanding the article into a tridimensional structure while being worn by the user. The means is activated by body fluids and comprises a sheet of compressed regenerated cellulose sponge in EP 96106721.2; the means is an expanding layer comprising apertures in EP 96106723.8 and incisions in EP 96106724.6.

These absorbent articles are capable of providing an anatomically shaped configuration for a closer body contact which is achieved during the use upon activation by absorbed body fluids, and are comfortable for the wearer and easy to produce and to package. Their performances can be improved in terms of control of the odours associated with absorbed body fluids.

The very close body contact achieved by this type of absorbent articles makes it desirable to provide these articles with an odour-control material. Due to their particular structure these articles are capable of staying very close to the wearer's body, and therefore they can acquire and absorb body fluids more rapidly and effectively than known absorbent articles; consequently, they can take even further advantage of the incorporation of an odour-control material than known absorbent articles.

Moreover, the closer body contact of these articles is per se capable of improving the effectiveness of any odour-control material since it brings the absorbed fluid in contact with the odour-control material immediately after the fluid itself has been released.

It is an object of the present invention to provide an absorbent article capable of providing an anatomically shaped configuration for a closer body contact which is achieved during the use upon activation by absorbed body fluids, while it is comfortable for the wearer and easy to produce and to package, and is capable of controlling the odours associated with absorbed body fluids.

### SUMMARY OF THE INVENTION

The present invention relates to disposable absorbent articles for wearing adjacent a body discharge area, which are substantially flat prior to use. The substantially flat disposable absorbent article has a longitudinal centreline and a lateral centreline orthogonal thereto that define longitudinal and lateral directions respectively, and a Z-direction that is orthogonal to both of them. The disposable absorbent article comprises a liquid pervious topsheet, a backsheet, preferably liquid impervious, joined to said topsheet, and an absorbent core intermediate the topsheet and the backsheet. The term "substantially flat", as used herein, refers to articles that have their main extension in one plane in contrast to being shaped. The absorbent core comprises means for expanding the article into a tridimensional structure while being worn by a user, wherein the means is activated by body fluids. The absorbent article further comprises an odour-control material comprising one or more odour-control agents.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a top plan view of one embodiment of a sanitary napkin according to the present invention;
FIG. 2 is a cross-sectional view of the sanitary napkin shown in FIG. 1 as taken along a section line corresponding to the transverse centreline A-A;
FIG. 3 is a cross-sectional view similar to that of FIG. 2, showing the sanitary napkin expanded into a tridimensional structure after activation during wear;
FIG. 4 is a cross-sectional view of another embodiment of a sanitary napkin according to the present invention;
FIG. 5 is a cross-sectional view similar to that of FIG. 2, showing the sanitary napkin expanded into a tridimensional structure after activation during wear.

### DETAILED DESCRIPTION OF THE INVENTION

This invention relates to a disposable absorbent article which exhibits absorbency for bodily fluids, the protection of the user's garments from soiling, improved physical comfort to the user, which is easy to produce and to package and exhibits an odour controlling capacity towards the odours associated with absorbed body fluids. The disposable absorbent article is described below by reference to a sanitary napkin or catamenial. The term "sanitary napkin", as used herein, refers to an article which is worn by females adjacent to the pudendal region and which is intended to absorb and contain the various body fluids which are discharged from the body (e.g., vaginal discharges, menses, and/or urine) and which is intended to be discarded after a single use. The disposable absorbent article is substantially flat prior to use.

The term "substantially flat", as used herein, refers to articles that have their main extension in one plane in contrast to being shaped. In a preferred embodiment a substantially flat article will have an absorbent core of constant thickness or, at least, will have an absorbent core that is not shaped in a direction which is orthogonal to the absorbent core itself. This does not exclude a general curvature of the absorbent core. It will be apparent to the man skilled in the art to which extent products can deviate from absolute flat shape and still benefit from the during the use shaping according to the present invention.

Sanitary napkins with longitudinal side cuffs, which may be optionally elasticated, and sanitary napkins with a moderate curvature are therefore within the scope of the present invention, provided that their absorbent core is not shaped prior to use in a direction that is orthogonal to the absorbent core itself.

The term "use", as used herein, refers to the period of time that starts when the absorbent article is actually put in contact with the anatomy of the user.

The terms "joined" or "affixed", as used herein, encompasses configurations whereby a first member is directly connected to a second member and configurations whereby a first member is indirectly connected to a second member by connecting the first member to intermediate members which in turn are connected to the second member.

Interlabial devices which reside partially within and partially external of the wearer's vestibule are also within the scope of this invention. As used herein, the term "pudendal" refers to the externally visible female genitalia and is limited to the labia majora, the labia minora, the clitoris, and the vestibule.

The development of unpleasant odours from body fluids as sweat, menstrual blood, vaginal discharges, or urine, are basically due to two different causes: a) malodorous chemical compounds already contained in the body fluids; and b) malodorous chemical compounds produced by the bacterial metabolism when the bacteria come in contact with body fluids for a prolonged period of time.

Means for controlling the odours associated with body fluids and their incorporation in disposable absorbent articles are widely known in the art, and various odour-control agents have been disclosed in the literature.

Different classes of odour-control agents are known in the art according to their different mechanisms of action. Disposable absorbent articles can comprise only one odour-control agent, or combinations of various odour-control agents, optionally belonging to different classes and therefore performing different actions for the control of unpleasant odours associated with body fluids.

A first class of odour-control agents is constituted by those compounds that interfere with the bacterial metabolism, in order to avoid or to reduce the production of malodorous metabolites from the body fluids; such agents can be bactericides or bacteriostats and are typically available as water-soluble compounds.

A second class of odour-control agents comprises those compounds, typically in particulate form, that are capable of adsorbing within their structure the odoriferous substances, both those already present in the body fluids as such and those produced by the bacterial metabolism.

Another class of odour-control agents comprises perfumes that essentially mask the unpleasant odours; moisture-activated encapsulated perfume particles can also be used, in which the perfumes are released only when the material is wetted, to provide their action during the use of the product, and to optionally avoid interaction with other odour-absorbing agents before the product is used, if such a combination is actually used as the odour-control material.

Any usual odour-control material can be used in the present invention, in conjunction with the means for expanding the article into a tridimensional structure while being worn by the user, to provide the article with the further benefit of controlling the odours associated with absorbed body fluids.

The odour-control agent employed in the practice of the present invention can be for example a water-soluble antibacterial compound. Such compounds include, for example, halogenated phenylene compounds (U.S. Patent 3,093,546), periodic acids (U.S. Patent 3,804,094), various copper compounds, especially copper acetate (U.S. Patent 4,385,632), various quaternary ammonium salts, which are well known for their antibacterial properties, e.g. cetyl pyridinium chloride, and the like. Alternatively, antibacterial compounds can be used conjointly with various particulate materials which, in use and in the presence of moisture, release the antibacterial agent. Zeolite materials, such as zeolites which are bactericidal by virtue of having absorbed therein and thereon various bactericidal cations such as copper, silver and zinc, can be advantageously used in the practice of this invention (U.S. Patent 4,525,410). In a preferred mode, the odour-control agent is a water-insoluble particulate odour-absorbing material such as chlorophyll particles, activated carbon granules, charcoal, ion exchange resin (Japanese 87019865), activated alumina, and absorbent zeolite materials, including the well known "molecular sieve" zeolites of the type A and X and the zeolite materials marketed under the trade name ABSCENTS by the Union Carbide Corporation and UOP, and which are typically available as a white powder in the 3-5 micron particle size range.

The odour-control materials used in the present invention can also comprise other compounds such as cyclodextrin, chelating agents, parabens, chitin, pH buffered materials, silica gel, hydrogel-forming absorbent gelling materials, and the like. Some partially neutralized hydrogel-forming absorbent gelling materials, such as polyacrylate gelling material and acrylate grafted starch gelling material can be also used, preferably in combination with other odour-control agents.

It is to be understood that the odour-control material employed in the practice of the present invention is not, simply, the odour-control agent, per se, added to the absorbent structure. Rather, the odour-control material comprises any combination of odour-control agents and, optionally, of other materials such as binders and/or substrates. The odour-control agent, on the other hand, is the specific odour-control compound.

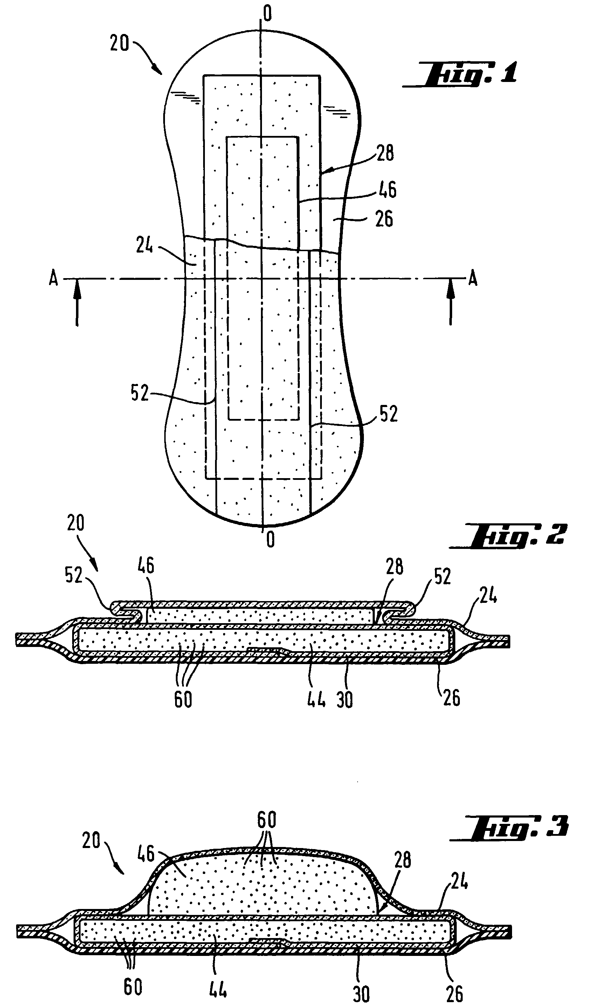

In FIGS. 1 and 2, one preferred embodiment of a sanitary napkin 20 of the present invention is shown. Fig. 1 is a plan view of the sanitary napkin 20 of the present invention in its flat-out state prior to use with portions of the structure being cut-away to more clearly show the construction of the sanitary napkin 20 and with the portion of the sanitary napkin 20 which faces or contacts the wearer oriented towards the viewer. As shown in FIGS. 1 and 2, the sanitary napkin 20 preferably comprises a liquid pervious topsheet 24, a liquid impervious backsheet 26 joined to the topsheet 24, and an absorbent core 28 intermediate the topsheet 24 and the backsheet 26; the absorbent core 28 comprises means, preferably an expanding layer 46, for expanding the sanitary napkin into a tridimensional structure while being worn by a user.

In a preferred embodiment of the present invention illustrated in FIGS. 1 to 3, the absorbent core 28 comprises the expanding layer 46 and a separate, substantially non expanding absorbent element 44 joined together and in face to face relationship to each other, the expanding layer 46 being positioned between the topsheet 24 and the absorbent element 44.

The absorbent element 44 and the expanding layer 46 may be associated in any suitable manner to form the absorbent core 28. Suitable manners include, but are not limited to, associating the absorbent element 44 and the expanding layer 46 with adhesives such as by spray-gluing or by applying lines or spots of adhesive between them. Alternatively, or additionally, the association between the layers may be achieved by fibre entanglement or by a plurality of discrete fusion bonds.

Alternatively, the expanding layer 46 may constitute the entire absorbent core 28.

The absorbent capacity of the absorbent core 28 should be compatible with the intended body fluid loading for the sanitary napkin 20. Further, the overall absorbent capacity of the absorbent core 28 may be varied to accommodate wearers ranging in the expected amount of body fluid volume. For instance, a different absorbent capacity may be utilized for sanitary napkins intended for day time use as compared with those intended for night time use, or for sanitary napkins intended for use by teenage females as compared with those intended by more mature women.

The sanitary napkin 20 has two surfaces, a body contacting or facing surface, and a garment facing or contacting surface. The absorbent core 28 has corresponding body facing and garment facing surfaces. The sanitary napkin 20 has two centrelines, a longitudinal centreline O-O and a transverse centreline A-A orthogonal thereto. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral", as used herein, are interchangeable, and refer to a line, axis, or direction which lies within the plane of the sanitary napkin 20 and is generally perpendicular to the longitudinal direction. The Z-direction is orthogonal both the longitudinal and lateral directions of the sanitary napkin 20 and extends outwardly from the plane of the sanitary napkin 20, which is defined by the longitudinal centreline O-O and the lateral centreline A-A. The term "longitudinally oriented" refers to a direction ±45 degrees of the longitudinal direction in the plane of the sanitary napkin 20; the term "laterally oriented" refers similarly to any other direction in the plane of the sanitary napkin 20.

The long edges of the sanitary napkin 20, which are aligned with the longitudinal centreline O-O, are the longitudinal side margins of the sanitary napkin 20. The ends of the sanitary napkin 20 joining the longitudinal side margins are the transverse ends of the sanitary napkin 20. Collectively the longitudinal side margins and transverse ends of the sanitary napkin 20 define its periphery. Similarly, the absorbent core 28 of the sanitary napkin 20 has a periphery defined by alternatively disposed longitudinal side margins and transverse ends.

Tridimensional structures of the sanitary napkin 20 are those in which the sanitary napkin structure is caused to expand, at least partially, in the Z-direction, in order to more closely conform to the user's anatomy. Said expansion preferably takes place in a direction that goes from the garment facing surface towards the body facing surface of the sanitary napkin 20. Particularly preferred are tridimensional structures with a convex upward configuration that are inclusive of, but not limited to, inverted U-shapes or inverted V-shapes, with "convex upward configuration" being meant a structure of the sanitary napkin that is convex on its body facing surface. With these configurations the cross-sectional contour of the central portion of the sanitary napkin more closely matches the labia of the typical wearer.

The topsheet 24 is compliant, soft feeling, and non-irritating to the wearer's skin. Further, the topsheet 24 is liquid pervious, permitting liquid to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core.

The topsheet of the present invention is preferably capable of expanding as the sanitary napkin 20 expands in a tridimensional structure upon absorption of body fluids. This may be achieved when the topsheet is made of a material that is intrinsically extensible under the forces exerted by the expanding layer 46. In a preferred embodiment illustrated in FIGS. 1 and 2 the topsheet 24 is provided with two pleats or folds 52 symmetrically positioned at both sides of the longitudinal centreline O-O and substantially parallel to it. As shown in FIG. 2 the topsheet 24 in each pleat or fold 52 is folded twice on itself toward the longitudinal side margins of the sanitary napkin 20. A single pleat or fold or, alternatively, more than two folds may be also comprised in the topsheet 24 without departing from the scope of the present invention; the pleats or folds may be generally longitudinally or laterally oriented.

The backsheet 26 is impervious to liquids (e.g., menses and/or urine) and is preferably manufactured from a thin plastic film, although other flexible liquid impervious materials can also be used. In use, the backsheet 26 is interposed between the absorbent core 28 and the user's undergarments. The function of the backsheet 26 is to prevent exudates which may be expelled from or which inadvertently bypass the absorbent core 28 from contacting and soiling the user's undergarments. The backsheet 26 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. The backsheet 26 is preferably embossed and/or matte finished to provide a more clothlike appearance.

In a preferred embodiment of the present invention the sanitary napkin 20 is also provided with a panty fastening means, not shown in the figures for clarity, which provides means to attach the article to an undergarment. For example the panty fastening means may comprise a mechanical fastener such as hook and loop fasteners such as marketed under the tradename VELCRO, snaps or holders. Alternatively, the sanitary napkin 20 is fastened to the undergarment by means of panty fastening adhesive on the backsheet 26. The panty fastening adhesive provides a means for securing the sanitary napkin 20 to the panty and preferably a means for securing the sanitary napkin 20 when soiled to the fold and wrap package for convenient disposal. Typically, at least a portion of the garment facing surface of the backsheet 26 is coated with adhesive to form the panty fastening adhesive. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

The panty fastening adhesive is typically applied to the backsheet by slot coating or spraying in various distribution patterns, such as e.g. continuous or discontinuous strips, intermittent dots, random patterns spirals.

The panty fastening adhesive is typically covered with a removable release paper or film in order to prevent the adhesive from drying out or adhering to another surface other than the panty prior to use. Any commercially available release paper or film may be used. Suitable examples include BL 30MG-A SILOX EI/O and BL 30 MG-A SILOX 4 P/O available from Akrosil Corporation.

If present, as illustrated in FIGS. 1 to 3, the substantially non expanding absorbent element 44 of the absorbent core 28 can comprise any absorbent means which is generally compressible, resilient, non-irritating to the wearer's skin and capable of absorbing and containing body fluids. The absorbent element 44 may be manufactured from a wide variety of liquid absorbent materials commonly used in disposable sanitary napkins, and other disposable absorbent articles. Examples of suitable absorbent materials include comminuted wood pulp (which is generally referred to as airfelt), creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et al. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlaid materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993 (P&G Case 5051)), absorbent sponges, synthetic staple fibres, polymeric fibres, hydrogel-forming polymer gelling agents, peat moss, or any equivalent materials or combinations of materials. Suitable absorbent cores comprising foams are described in European Applications 0 598 833, 0 598 823 and 0 598 834.

In the embodiment illustrated in FIGS. 1 to 3, the substantially non expanding absorbent element 44 of the absorbent core 28 comprises an absorbent layer 30 made of a thermally bonded airlaid material longitudinally folded twice on itself and comprising particles of absorbent gelling material therebetween, which are not shown for clarity.

As shown in FIGS. 1 and 2 the absorbent core 28 comprises an expanding layer 46 for expanding the sanitary napkin 20 into the desired tridimensional structure while the sanitary napkin 20 is being worn. In an embodiment illustrated in FIGS. 1 to 3 the expansion and the final shaping of the sanitary napkin 20 into the tridimensional structure is provided by the swelling, substantially in Z-direction, of the material that constitutes the expanding layer 46 and that is activated during wear by the absorption of body fluids.

The expanding layer 46 can comprise any material that is capable of such swelling in order to shape the sanitary napkin 20 into the desired tridimensional structure.

After the absorption of body fluids and the subsequent swelling, the material of the expanding layer 46 must be soft, compliant, conformable and resilient. It must be compressible such that it will deform under the relatively small forces that are experienced during normal use. In addition to be compressible, the material of the expanding layer 46 must be flexible and conformable after swelling so it can provide improved fit through the topsheet 24 into and around the wearer's labia and perineum when the tridimensional structure is formed during the wearing time. The ability to follow the topography of the anatomy will provide intimate contact with the exposed genitalia of the female user. This helps provide better fluid transfer from the user into the expanding layer 46. While these characteristics of the expanding layer 46 allow for improved fit, they also cause the product to be both soft and comfortable for the wearer.

It is preferred that the expanding layer 46 forms at least part of the body facing surface of the absorbent core 28. In the embodiment illustrated in FIGS. 1 and 2 the expanding layer 46 is positioned over the absorbent element 44, in face to face relationship with it; it is rectangular and preferably narrower and shorter than the absorbent element 44, as illustrated in FIG. 1, being centered about both the longitudinal and transverse centrelines O-O and A-A. As an alternative, different shapes are also possible for the expanding layer 46, e.g. an hourglass shape.

The expanding layer 46 of the absorbent core 28 has a body facing surface and a garment facing surface, and can further comprise, on at least one of its body facing surface or its garment facing surface, incisions as described in European application EP 96106724.6, or apertures as described in European application EP 96106723.8, both application filed on 29 April 1996, or any combination thereof.

In a preferred embodiment of the present invention the expanding layer 46 comprises a sheet of compressed regenerated cellulose sponge.

The regenerated cellulose sponge that preferably constitutes the expanding layer 46 is a material that is known in the art; examples of suitable materials are described in U.S. Patent No. 3,954,493, in French Patent Application FR-A-2,203,827, and in European Patent EP-B-0 293 208. The regenerated cellulose sponge is a sponge of a material containing a cellulose skeleton. Examples of such sponges include, in addition to sponges consisting of cellulose itself, sponges consisting of a cellulose derivative as viscose, a cellulose ether and a cellulose ester, and sponges consisting of mixtures of those materials.

By way of example only, a regenerated cellulose sponge may be prepared from a mixture of a viscose solution with reinforcing fibres and a porogenic compound, e.g. crystals of sodium sulphate decahydrate or of another alkali metal salt with a high content of crystallized water, the final pore dimension being related to that of the salt crystals. The viscose solution may be extruded through an extrusion die of the desired section, then let coagulate. The material is washed with water after regeneration in order to eliminate the salt and other possible soluble compounds, then it is dried and, if necessary, compressed to the desired density.

The compressed regenerated cellulose sponge has a network structure that contains air bubbles created by the elimination of the sodium sulphate crystals.

The compressed regenerated cellulose sponge material is available in various forms, e.g. in layers or sheets of different densities, thicknesses and basis weights; dry densities values for the compressed material used in the present invention are from 0.1 g/cc to 1 g/cc, while thicknesses may range from 0.2 mm to 5 mm.

The swelling upon liquid absorption of the compressed regenerated cellulose sponge material that forms the expanding layer 46 creates a void volume that does not collapse in wet conditions and therefore enables the material to rapidly acquire further releases of fluid and to transmit them to the underlying absorbent element 44 of the absorbent core 28.

In the embodiment illustrated in FIGS. 1 to 3 the total absorbent capacity of the sanitary napkin 20 is provided for by an absorbent core 28 that comprises and expanding layer 46 made of a sheet of compressed regenerated cellulose sponge, and a substantially non expanding absorbent element 44.

In a preferred embodiment of the present invention, which is illustrated in FIGS. 1 and 2, the absorbent core 28 comprises an expanding layer 46 constituted by a sheet of compressed regenerated cellulose sponge with a dry density of 0.5 g/cc and a thickness of 2 mm is positioned above the absorbent core 28 in face to face relationship with it. The absorbent layer 30 is 207 mm long and 64 mm wide, and the sheet 46 of compressed regenerated cellulose sponge is 125 mm long and 30 mm wide, being centered about both longitudinal and transverse centrelines O-O and A-A of the sanitary napkin 20. Suitable sheets of compressed regenerated cellulose sponge may be those produced by Spontex France.

The compressed regenerated cellulose sponge sheet that preferably constitutes the expanding layer 46 is capable of absorbing body fluids quickly with a large increase in its volume, generally from about 2 to 20 times, and usually from 5 to 15 times its volume at the time of the compression. The volume increase substantially corresponds to a swelling in the direction of the compression, that is in the Z-direction in the sanitary napkin 20.

The sanitary napkin 20 is produced and packaged as a conventional flat product, as illustrated in FIGS. 1 and 2. After the sanitary napkin 20 has been worn, as soon as the absorbed body fluids come in contact with the expanding layer 46, this will begin to swell in Z-direction increasing its thickness, as can be seen in FIG. 3. The topsheet 24 follows the swelling of the expanding layer 46 by straightening out the pleats or folds 52, therefore increasing its width without restraining the swelling.

The swelling of the compressed regenerated cellulose sponge sheet that constitutes the expanding layer 46 takes place only upon activation by the absorbed fluid, that is only during the use of the sanitary napkin 20 and in close contact with the user's anatomy; the formation of the tridimensional structure can therefore achieve a much better fit with the anatomy of the user. Moreover, the swelling of the compressed regenerated cellulose sponge sheet 46 may start where it is actually reached by the fluid first; the formation of the tridimensional structure may also fit, therefore, the different possible ways in which the body fluids may be released by various users.

The expanding topsheet 24 also provides a comfortable contact with the user's anatomy, without restraining the expansion of the sanitary napkin 20 into the desired tridimensional structure upon activation by body fluids.

The sanitary napkin of the present invention is flat prior to use, and can be therefore manufactured and packaged more easily than a conventional elasticated or pre-formed article. Since the tridimensional structure is formed only during the use, the sanitary napkin of the present invention is also easier to wear.

The expanding element 46 for expanding the sanitary napkin 20 into a tridimensional structure during wear may be comprised in the sanitary napkin 20 in any other suitable position and/or orientation in order to get the desired tridimensional structure, in particular, the expanding element 46 can form at least part of the garment facing surface of the absorbent core 28.

The sanitary napkin 20 of the present invention further comprises an odour-control material 60 for controlling unpleasant odours associated with absorbed body fluids.

Various body fluids contain malodorous chemical compounds including acyclic and cyclic amines, aldehydes, fatty acids, and sulfur containing compounds such as sulfides. Vaginal discharges and used sanitary napkins can contain many malodorous chemical compounds, for example, trimethylamine, pyridine, furaldehyde, isovaleric acid, and methyl mercaptan. The particular malodorous compounds which will be absorbed by various absorbent articles will vary depending upon the person who is wearing the absorbent article and the type of body fluid absorbed, i.e., urine, menstrual fluid, vaginal discharges, perspiration, milk, etc. For feminine pads, such as sanitary napkins or pantiliners, the length of time the article is worn, the quantity of fluid which is absorbed and the exposure of the pad to different body fluids will determine which odours can be emitted by the absorbent article.

Any known odour-control agent or any combination thereof that can be suitably included in a disposable absorbent article, including other materials such as binders and/or substrates, can be comprised in the absorbent article of the present invention as the odour-control material.

The odour-control material can be incorporated into the absorbent article by methods known in the art, for example layered on or into the absorbent core or mixed within the absorbent core. The odour-control material can be incorporated between two layers of cellulose tissue, optionally the material can be bonded between the two layers with, for example, a hot melt adhesive or any suitable bonding system.

The odour-control material is typically comprised in the absorbent core 28 of the sanitary napkin 20 of the present invention.

In an embodiment of the present invention illustrated in FIGS. 1 to 3 the sanitary napkin 20 can comprise the odour-control material 60 in particle form within the structure of the substantially non expanding absorbent element 44; the particles of the odour-control material 60 are uniformly distributed between the folded layers of the absorbent layer 30, together with the particles of absorbent gelling material that are not shown for clarity.

The odour-control material 60 can be a combination of silica gel, zeolite and absorbent gelling material particles.

Preferably, an odour-control material can be also included in the expanding layer 46; this odour-control material can be of the same type, or of a different type, with respect to that included in the substantially non expanding absorbent element 44 of the absorbent core 28.

When the expanding layer 46 is constituted by a sheet of compressed regenerated cellulose sponge, as it is preferred, the odour-control material may be included within the sponge structure with any known method, e.g. particles of the odour-control material can be distributed within the network structure of the sponge, as disclosed in the U.S. Patent 5,441,742.

It has been found that the odour control material can be incorporated into the absorbent articles according to the present invention in an amount that ranges from 20 g/m² to 400 g/m², preferably from 100 g/m² to 300 g/m², more preferably from 150 g/m² to 250 g/m², with reference to the total surface area of the absorbent article in its flat state prior to use; the weight of the odour-control material that can be used in various absorbent articles can be readily determined by the skilled person bearing in mind the size and the type of the absorbent article.

In use, the swelling of the expanding layer 46 that takes place upon activation by the absorbed body fluid provides the absorbent article with a very close fit with the user's anatomy; the close body contact achieved in use by the absorbent article of the present invention improves the effectiveness of the odour-control material comprised into the absorbent article, especially when, as it is preferred, both the expanding layer 46 and the substantially non expanding layer 44 of the absorbent core 28 include an odour-control material.

The body fluid is in fact brought into contact with the odour-control material immediately after its release, as soon as it is absorbed within the body conforming structure of the absorbent article; this allows a better control of the odours associated with the absorbed body fluids both in terms of adsorption of the malodorous compounds already present in the absorbed body fluids, and in terms of prevention of the formation of other malodorous compounds, according to the type of odour-control agents comprised in the odour-control material. At the same time, the close fit of the absorbent article of the present invention reduces the possibility that malodorous substances can escape from the absorbent article itself during the wearing time.

In an alternate embodiment of the present invention, the sanitary napkin 20 may have two flaps (not shown), each of which are adjacent to and extend laterally from the side edge of the absorbent core. The flaps are configured to drape over the edges of the wearer's panties in the crotch region so that the flaps are disposed between the edges of the wearer's panties and the wearer's thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment facing surface so that the flaps can be folded back under the panty and attached to the garment facing side of the panty. In this way, the flaps serve to keep the sanitary napkin properly positioned in the panty.

The flaps may be constructed of various materials including materials used for the topsheet 24, backsheet 26, combinations thereof, and may be a laminate having tissue in the centre. Further, the flaps may be a separate element attached to the main body of the sanitary napkin 20 or can comprise extensions of the topsheet 24 and/or backsheet 26. It is recommended, however, that the flaps have a liquid impervious backsheet to prevent body fluids which reach the flaps from soiling the edges of the wearer's panties.

Preferred flaps that are suitable or adaptable to the sanitary napkin 20 of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, the sanitary napkin 20 may comprise components that naturally wrap the sides of a wearer's panties. Sanitary napkins having components that naturally wrap the sides of a wearer's panties suitable for use with the sanitary napkin 20 of the present invention are disclosed in U.S. Patent Application Serial No. 08/096,121 entitled "Absorbent Article having Panty Covering Components that Naturally Wrap the Sides of Panties", filed July 22, 1993, in the names of Lavash, et al and U.S. Patent Application Serial No. 08/277733 entitled "Absorbent Articles Having Undergarment Covering Components with Zones of Extensibility", filed July 20, 1994, in the names of Weinberger, et al.

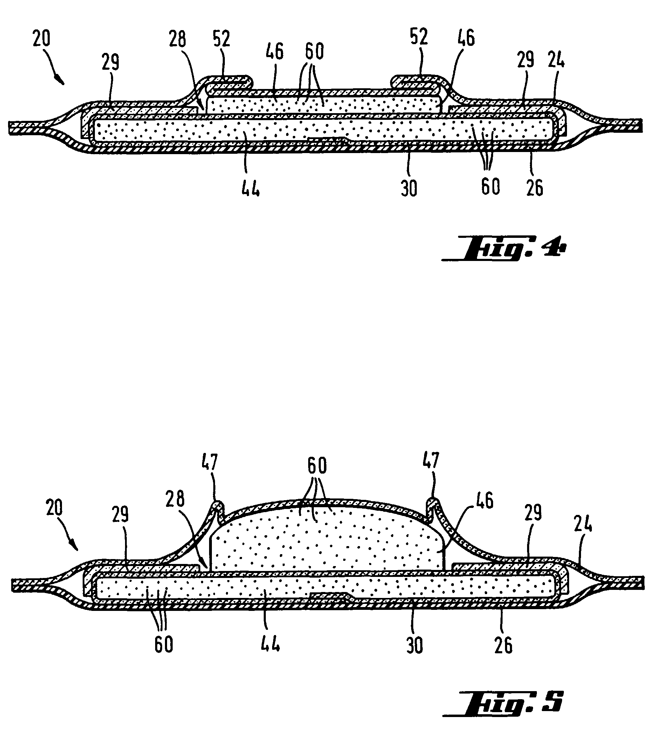

In an alternate embodiment illustrated in FIG. 4 a sanitary napkin 20 similar to that illustrated in FIGS. 1 and 2 further comprises an acquisition layer or secondary topsheet 29 positioned between the topsheet 24 and the absorbent core 28. Preferably the acquisition layer 29 does not completely overlie the absorbent core 28; in the embodiment illustrated in FIG. 4 the acquisition layer 29 does not cover the expanding layer 46 that is therefore capable of receiving the body fluids directly through the topsheet 24. As illustrated in FIG. 4 the acquisition layer 29 has a discontinuous surface comprising a window which is slightly longer and wider than the expanding layer 46; therefore the acquisition layer 29 is actually comprised between the topsheet 24 and the substantially non expanding absorbent element 44 of the absorbent core 28. Alternate configurations may also be possible, e.g. the acquisition layer 29 may comprise two narrow strips longitudinally oriented and positioned over the absorbent element 44 of the absorbent core 28 at both sides of the expanding layer 46. Alternatively, the acquisition layer 29 can be comprised between the absorbent core 28 and the backsheet 26; further, the acquisition layer 29 can be comprised between the expanding layer 46 and the absorbent element 44 in an embodiment similar to that illustrated in FIG. 2.

The acquisition layer 29 may serve several functions including improving wicking of body fluids that may escape laterally from the expanding layer 46, or, alternatively, that may reach the acquisition layer 29 directly, over and into the absorbent element 44 of the absorbent core 28. By improving wicking of body fluids, the acquisition layer 29 provides a more even distribution of the body fluids throughout the absorbent core 28.

The acquisition layer 29 preferably comprises materials that are capable of acquiring liquid very fast, and subsequently release it to contiguous layers with substantially no retention capacity.

The acquisition layer 29 may be comprised of several different materials including nonwoven or woven webs of synthetic fibres including polyester, polypropylene, or polyethylene; natural fibres including cotton or cellulose; blends of such fibres; or any equivalent materials or combinations of materials. Examples of sanitary napkins having an acquisition layer and a topsheet are more fully described in U.S. Pat. No. 4,950,264 issued to Osborn and U.S. Pat. Application Serial No. 07/810,774, "Absorbent Article Having Fused Layers", filed December 17, 1991 in the names of Cree, et al.

The topsheet 24, the acquisition layer 29 and the absorbent core 28 may also be associated in any suitable manner, in order to insure proper fluid transfer between them. In a further alternative embodiment that is not illustrated the acquisition layer 29 may be interposed between the topsheet 24 and the underlying absorbent core 28 comprising the expanding layer 46; the acquisition layer 29 must be left free to follow the expansion of the expanding layer 46 upon absorption of liquid, without restraining its swelling.

As illustrated in FIG. 4, the pleats or folds 52 are positioned at both sides of the longitudinal centreline O-O and substantially parallel to it, but in each pleat or fold 52 the topsheet 24 is folded twice on itself toward the longitudinal centreline O-O of the sanitary napkin 20. During the swelling of the expanding layer 46 upon fluid absorption the straightening out of the pleats or folds 52 forms a sort of longitudinally oriented side cuffs 47 that provide a better seal against side leakage, as illustrated in FIG. 5; the side cuffs 47 may still be present when the swelling of the expanding layer 46 is completed if the overall width of the topsheet 24 is slightly higher than that which would be necessary to follow the complete swelling of the expanding layer 46.

Although the disposable absorbent article of the present invention has been described with reference to a sanitary napkin, it can be used beneficially in the context of other disposable absorbent articles such as panty liners, incontinence articles and diapers. The disposable absorbent article may thus also have all those features and parts which are typical for products in the context of their intended use.

## Claims

1. A disposable absorbent article for wearing adjacent a body discharge area, said article having a longitudinal centreline and a lateral centreline orthogonal thereto and defining longitudinal and lateral directions respectively, said article further having a Z-direction which is orthogonal to both said longitudinal and lateral directions, said article comprising a liquid pervious topsheet, a backsheet joined to said topsheet, an absorbent core intermediate said topsheet and said backsheet, said absorbent article being substantially flat prior to use, said absorbent core comprising means for expanding said article into a tridimensional structure while being worn by a user, said means being activated by body fluids, said absorbent core comprising a body facing surface, said absorbent article characterized in that it further comprises an odour-control material comprising one or more odour-control agents.

2. A disposable absorbent article according to claim 1, characterized in that said means for expanding said article provides said article with a substantially convex upward configuration.

3. A disposable absorbent article according to any preceding claim, characterized in that said means for expanding said article upon activation by body fluids expands substantially in said Z-direction to provide said tridimensional structure.

4. A disposable absorbent article according to any preceding claim, characterized in that said topsheet is capable of expanding as said absorbent article expands upon activation by body fluids.

5. A disposable absorbent article according to any preceding claim, characterized in that said means for expanding said article comprises a sheet of compressed regenerated cellulose sponge having a dry density of 0.1÷1 g/cc.

6. A disposable absorbent article according to claim 6, characterized in that said sheet of compressed regenerated cellulose sponge has a thickness of 0.2÷5 mm.

7. A disposable absorbent article according to any preceding claim, characterized in that said odour-control agent is selected from the group consisting of: antibacterial compounds, zeolites, activated carbon, charcoal, silica gel, siliceous molecular sieves, activated alumina, cyclodextrin, cheating agents, chlorophyll, parabens, chitin, inorganic salts, ion exchange resins, acidic, basic or neutral buffer systems, absorbent gelling materials, perfumes, and combinations thereof.

8. A disposable absorbent article according to any preceding claim, characterized in that said odour-control material is comprised with a basis weight that ranges from 20 g/m² to 400 g/m², preferably from 100 g/m² to 300 g/m², more preferably from 150 g/m² to 250 g/m².

9. A disposable absorbent article according to any preceding claim, characterized in that said odour-control material comprises a combination of silica gel, absorbent gelling material and zeolite.

10. A disposable absorbent article according to any preceding claim, characterized in that said disposable absorbent article is a sanitary napkin or a pantiliner.
